# FASCICULE DE BREVET EUROPEEN

(11) **EP 1 292 608 B1**
(45) Date de publication et mention de la délivrance du brevet: **21.07.2004**
(21) Numéro de dépôt: 01936599.8
(22) Date de dépôt: 21.05.2001
(51) Int. Cl.: C07K 5/06, A61K 38/55, A61K 7/48

(54) **NOUVEAUX COMPOSES DE LA FAMILLE DES N-ACYLAMINO-AMIDES, COMPOSITIONS LES COMPRENANT, ET UTILISATIONS**
NEUE VERBINDUNGEN DER FAMILIE VON N-ACYLAMINOAMIDEN, SIE ENTHALTENDE ZUSAMMENSETZUNGEN UND VERWENDUNGEN
NOVEL COMPOUNDS OF THE N-ACYLAMINO-AMIDE FAMILY, COMPOSITIONS COMPRISING SAME, AND USES

(30) Priorité: 08.06.2000 FR 0007344
(43) Date de publication de la demande: 19.03.2003
(73) Titulaire: L'OREAL, 75008 Paris (FR)
(72) Inventeur: DALKO, Maria, F-91190 Gif sur Yvette (FR); MAHE, Yann, F-91390 Morsang-sur-Orge (FR); BRETON, Lionel, F-78000 Versailles (FR)
(74) Mandataire: Dodin, Catherine
(86) Numéro de dépôt international: PCT/FR2001/001559
(87) Numéro de publication internationale: WO 2001/094381

(56) Documents cités:
- EP-A- 0 498 728
- WAKI, MICHINORI ET AL: "Peptide synthesis using the four-component condensation (Ugi reaction)" J. AM. CHEM. SOC. (1977), 99(18), 6075-82, 1977, XP002165353

## Description

La présente invention a trait à de nouveaux composés de la famille des N-acylamino-amides, à leur utilisation notamment en cosmétique ou en pharmacie, et aux compositions les comprenant.

La peau humaine est constituée de deux compartiments à savoir un compartiment superficiel, l'épiderme, et un compartiment profond, le derme.
L'épiderme humain naturel est composé principalement de trois types de cellules qui sont les kératinocytes, très majoritaires, les mélanocytes et les cellules de Langerhans. Chacun de ces types cellulaires contribue par ses fonctions propres au rôle essentiel joué dans l'organisme par la peau.
Le derme fournit à l'épiderme un support solide. C'est également son élément nourricier. Il est principalement constitué de fibroblastes et d'une matrice extracellulaire composée elle-même principalement de collagène, d'élastine et d'une substance, dite substance fondamentale, composants synthétisés par le fibroblaste. On y trouve aussi des leucocytes, des mastocytes ou encore des macrophages tissulaires. II est également traversé par des vaisseaux sanguins et des fibres nerveuses.

On sait que lors d'un stress cutané superficiel, qui peut notamment être d'origine chimique, physique ou bactérienne, les kératinocytes des couches superficielles de l'épiderme libèrent des médiateurs biologiques qui possèdent la capacité d'attirer certaines cellules infiltrantes de la peau, elles-mêmes responsables de l'entretien d'une irritation locale transitoire.
Parmi les médiateurs biologiques pouvant être produits par les kératinocytes ainsi stressés, on citera les chimiokines qui sont des cytokines chimioattractives responsables du recrutement de leukocytes sur les sites inflammatoires, dont l'interleukine 8 (IL-8) qui est plus particulièrement responsable du recrutement des neutrophiles.
Ces cellules infiltrant les zones irritées ou agressées libèrent alors des enzymes parmi lesquelles on peut citer l'élastase leucocytaire.
Sous l'action de cette enzyme notamment, les fibres élastiques de soutien extracellulaire du tissu conjonctif peuvent être dégradées, et entraîner ainsi une diminution de l'élasticité de la peau.
Il est même par ailleurs connu qu'en synergie avec la cathepsine G, l'élastase leucocytaire peut dissocier l'intégrité de l'épiderme en élargissant les espaces intercellulaires interkératinocytaires.
Ainsi, à long terme, la somme des micro-stress cutanés superficiels, par exemple générés par une exposition prolongée aux UV ou par des agents irritants, peut entraîner une perte plus ou moins accélérée de l'élasticité naturelle de la peau. Le réseau formé par les fibres élastiques du tissu conjonctif sous-jacent et des espaces extracellulaires peut alors progressivement être destructuré. Il s'en suit un vieillissement accéléré de la peau (peau ridée et/ou moins souple) via l'altération du réseau élastique dermique, ainsi qu'une accentuation des rides (rides plus profondes).

Par ailleurs, on sait que la solidité du derme est principalement assurée par les fibres de collagène. Ces fibres sont constituées de fibrilles scellées les unes aux autres, formant ainsi plus de dix types de structures différentes. La solidité du derme est en grande partie due à l'enchevêtrement des fibres de collagène tassées les unes contre les autres en tous sens. Les fibres de collagène participent à l'élasticité et à la tonicité de la peau et/ou des muqueuses.
Les fibres de collagènes sont constamment renouvelées mais ce renouvellement diminue avec l'âge ce qui entraîne un amincissement du derme. Cet amincissement du derme est également dû à des causes pathologiques comme par exemple l'hypersécrétion d'hormones corticoïdes, certaines pathologies ou encore des carences vitaminiques (cas de la vitamine C dans le scorbut). Il est également admis que des facteurs extrinsèques comme les rayons ultraviolets, le tabac ou certains traitements (Glucocorticoïdes, vitamine D et dérivés par exemple) ont également un effet sur la peau et sur son taux de collagène.
Bien que très résistantes, les fibres de collagène sont sensibles à certaines enzymes appelées collagénases. Une dégradation des fibres de collagène entraîne l'apparence de peau molle et ridée que l'être humain, préférant l'apparence d'une peau lisse et tendue, cherche depuis toujours à combattre.
Par ailleurs à la ménopause, les principales modifications concernant le derme sont une diminution du taux de collagène et de l'épaisseur dermique. Cela entraîne chez la femme ménopausée un amincissement de la peau et/ou des muqueuses. La femme ressent alors une sensation de "peau sèche" ou de peau qui tire et l'on constate une accentuation des fines rides et ridules de surface. La peau présente un aspect rugueux à la palpation. Enfin la peau présente une souplesse diminuée.

La présente invention a pour but de proposer une solution à ces différents problèmes, et notamment de proposer de nouveaux composés susceptibles d'être utilisées en cosmétique ou en pharmaceutique pour limiter le vieillissement de la peau, qu'il soit chronobiologique ou photo-induit, et notamment le vieillissement généré par une diminution de l'élasticité de la peau et/ou par une dégradation du collagène dans la structure des tissus.

Sans être tenu par la présente explication, on peut considérer que le fait d'apporter, au niveau des kératinocytes des couches superficielles de la peau, des composés susceptibles de ralentir l'activité de dégradation des fibres élastiques des espaces intercellulaires, peut permettre de diminuer ce phénomène de vieillissement accéléré de la peau, dû à des stress cutanés superficiels.

Certains composés appartenant à la famille des N-acylamino-amides sont connus dans l'art antérieur. A titre d'exemple au peut citer le document *J. Am. Chem.* Soc., 1977, 99(18) pp. 6075-82) qui décrit un procédé de synthèse des dérivés suivants :
- *N*-Acétylglycyl-*N*-(benzyl)-DL-valylglycine *tert-*butyl ester ;
- *N*-Acétylglycyl-*N*-(2-nitrobenzyl)-DL-valylglycine *tert*-butyl ester ;
- *N*-Acétylglycyl-*N*-(2,4-diméthyloxybenzyl)-DL-valylglycine *tert* butyl ester.

La présente invention a donc pour objet un composé de formule (1) telle que ci-après définie, à l'exception des dérivés suivants :
- *N*-Acétylglycyl-*N*-(benzyl)-DL-valylglycine *tert*-butyl ester ;
- *N*-Acétylglycyl-*N*-(2-nitrobenzyl)-DL-valylglycine *tert*-butyl ester ;
- *N*-Acétylglycyl-*N*-(2,4-diméthyloxybenzyl)-DL-valylglycine *tert*-butyl ester.

L'invention a également pour objet une composition comprenant, dans un milieu physiologiquement acceptable, au moins un tel composé de formule (I).
Un autre objet de l'invention est l'utilisation d'au moins un composé de formule (I) ou d'une composition le comprenant, pour la préparation d'un médicament destiné à traiter, de manière préventive ou curative, les signes du vieillissement de la peau du corps ou du visage, qu'il soit chronobiologique ou photo-induit, et notamment le vieillissement généré par une diminution de l'élasticité de la peau et/ou par une dégradation du collagène dans la structure des tissus.
Un autre objet de l'invention est l'utilisation d'au moins un composé de formule.(I) ou d'une composition le comprenant, pour la préparation d'un médicament destiné à traiter, de manière préventive ou curative, les rides et/ou ridules, la peau flétrie, le manque d'élasticité et/ou de tonus de la peau, l'amincissement du derme, la dégradation des fibres de collagène, la peau molle, la peau amincie; les dégradations internes de la peau consécutives à une exposition aux rayonnements ultra-violets.
Un autre objet de l'invention est l'utilisation d'a moins un composé de formule (I) ou d'une composition le comprenant, pour la péparation d'un médicament destiné à inhiber l'activité des élastases et/ou pour limiter et/ou combattre la dégradation des fibres élastiques.
Un autre objet de l'invention est un procédé de traitement cosmétique de la peau du corps ou du visage, y compris le cuir chevelu, dans lequel on applique sur la peau une composition cosmétique telle que définie ci-après.

Il a en effet été constaté que les composés de formule (I) présentaient une activité inhibitrice de l'activité des élastases, et qu'ils pouvaient donc être employés pour limiter et/ou combattre la dégradation des fibres élastiques.
Il s'en suit qu'ils peuvent être employés dans ou pour la préparation d'une composition, les composés ou la composition étant destinés à traiter, de manière préventive et/ou curative, les signes cutanés du vieillissement.

Par signes cutanés du vieillissement on entend toutes modifications de l'aspect extérieur de la peau dues au vieillissement qu'il soit chronobiologique et/ou photo-induit, comme par exemple les rides et ridules, la peau flétrie, le manque d'élasticité et/ou de tonus de la peau, l'amincissement du derme et/ou la dégradation des fibres de collagène ce qui entraîne l'apparence de peau molle et ridée; on entend également toutes les modifications internes de la peau qui ne se traduisent pas systématiquement par un aspect extérieur modifié, comme par exemple toutes dégradations internes de la peau, particulièrement des fibres d'élastine, ou fibres élastiques, consécutives à une exposition aux rayonnements ultra-violets.

Un avantage de la présente invention réside dans le fait que les composés de formule (I) peuvent aisément être préparés.

Les composés susceptibles d'être employés dans la présente invention répondent donc à la formule (1) suivante : 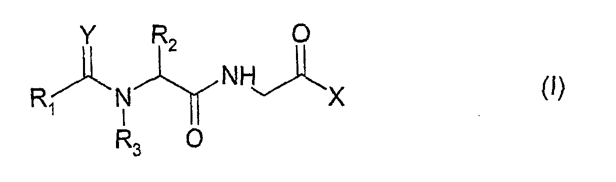 dans laquelle :
- le radical Y représente O ou S,
- le radical R1 représente :
   - (i) un atome d'hydrogène;
   - (ii) un radical hydrocarboné, linéaire, ramifié ou cyclique, saturé ou insaturé, ayant 1 à 18 atomes de carbone,
      éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR; -P(O)-(OR)₂; -SO₂-OR;
      avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
   - (iii) un radical choisi parmi les radicaux -OR; -NH₂; -NHR; -NRR'; -NH-COR; -COOR; -COR ;
      avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
      lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR";
      -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R2 représente un radical hydrocarboné, linéaire, ramifié ou cyclique, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR;
   avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R3 représente un radical choisi parmi ceux de formule (II) ou (III) :

   (II) -A-C₆H_{(5-y)}-B_{y}

   (III) -C₆H_{(5-y')}-B_{y'}
dans lesquelles :
- y est un entier compris entre 0 et 5 inclus, et y' est un entier compris entre 1 et 5 inclus;
- A est un radical divalent hydrocarboné, linéaire ou ramifié, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR;
   avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- B représente au moins un groupement, identique ou différent, choisi parmi -OH; -OR; -O-COR; -SH; -SR; -S-COR; -NH₂; -NHR; -NRR'; -NH-COR; -Halogène; -CN; -COOR; -COR; -NO₂; -SO₂-OR, ou représente un radical hydrocarboné, linéaire ou ramifié, saturé ou insaturé, ayant 1 à 18 atomes de carbone,
   éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR
   avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
   lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical X représente un radical choisi parmi -OH, -OR₄, -NH₂, -NHR₄, -NR₄R₅ , -SR₄, -COOR₄; -COR₄;
   avec R₄ et R₅ représentant, indépendamment l'un de l'autre, un radical hydrocarboné, linéaire, cyclique ou ramifié, saturé ou insaturé, ayant 1 à 6 atomes de carbone, éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné; lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
   lesdits radicaux R₄ et R₅ pouvant former ensemble avec N un cycle carboné à 5
ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR' ; -O-COR"; -SH;-SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné,
à l'exception des dérivés suivants :
- *N*-Acétylglycyl-*N*-(benzyl)-DL-valylglycine *tert-*butyl ester ;
- *N*-Acétylglycyl-*N*-(2-nitrobenzyl)-DL-valylglycine *tert*-butyl ester ;
- *N*-Acétylglycyl-*N*-(2,4-diméthyloxybenzyl)-DL-valylgiycine *tert-*butyl ester.

Sont également compris dans cette définition, les sels d'acide minéral ou organique desdits composés, ainsi que leurs isomères optiques, sous forme isolée ou en mélange racémique.

Par radical hydrocarboné, linéaire, cyclique ou ramifié, on entend notamment les radicaux de type alkyle, aryle, aralkyle, alkylaryle, alcényle, alcynyle.

Le groupement C₆H₅ présent dans le radical R3 doit être compris comme un groupement cyclique aromatique.

De préférence, le radical Y représente l'oxygène.

De préférence, le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué.
Notamment, les substituants peuvent être choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné.
Préférentiellement, le radical R1 représente un radical méthyle, éthyle, propyle ou isopropyle, éventuellement substitué par un groupement -OH ou -P(O)-(OR)₂ avec R représentant méthyle, éthyle, propyle ou isopropyle.

De préférence, le radical R2 représente un radical hydrocarboné, linéaire, ramifié
ou cyclique, saturé ou insaturé, ayant 1 à 12, notamment 1, 2, 3, 4, 5 ou 6, atomes de carbone, éventuellement substitué.
Notamment, les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R2 représente un radical méthyle, éthyle, propyle, isopropyle, n-butyle, ter-butyle ou isobutyle.

De préférence, le radical R3 représente un radical de formule -C₆H(_{5-y'})-B_{y'} pour lequel y' = 1, 2 ou 3; ou un radical de formule -A-C₆H(_{5-y})-B_{y} pour lequel y = 0, 1 ou 2.
De préférence, A est un radical divalent hydrocarboné, linéaire ou ramifié, saturé ou insaturé, ayant 1 à 12 atomes de carbone, éventuellement substitué.
Les substituants de A sont de préférence choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné.
De préférence, B représente au moins un groupement -OR; -NHR; -CN; -COOR; - COR ou représente un radical hydrocarboné choisi parmi un radical hydrocarboné, linéaire ou ramifié, saturé ou insaturé, ayant 1 à 12 atomes de carbone, éventuellement substitué.
Les substituants de B sont de préférence choisis parmi -Hal (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné.

Préférentiellement, le radical R3 représente un groupement choisi parmi l'une des formules suivantes : 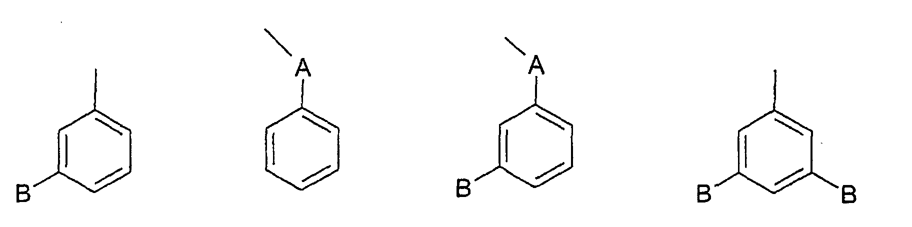 dans lesquelles A et B ont les significations ci-dessus.

Notamment, le radical divalent A peut être un méthylène, un éthylène, un propylène.

De préférence, le radical B représente au moins un groupement -OR; -NHR; -CN; - COOR; -COR pour lesquels R désigne un radical méthyle, éthyle, propyle ou isopropyle, ou représente un radical hydrocarboné choisi parmi un radical méthyle, éthyle, propyle ou isopropyle, substitué par un ou plusieurs halogènes, notamment chlore, brome, iode ou fluor, et préférentiellement totalement halogéné (perhalogéné), tel que perfluoré. On peut en particulier citer le radical perfluorométhyle (-CF3) comme tout particulièrement préféré.

De préférence, le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué.
Les substituants peuvent être choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.
Préférentiellement, le radical X représente un radical choisi parmi -OH, -OCH₃, -OC₂H₅, -O-C₃H₇ ou -OC₄H₉.

Parmi les composés particulièrement préférés, on peut citer :
- l'acide {2-[acétyl-(3-triftuorométhyt-phényl)-amino]-3-méthyt-butyrylamino} acétique,
- le {2-[acétyl-(3-trifluorométhyl-phényl)-amino]-3-méthyl-butyrylamino} acétate d'éthyle,
- l'acide [2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[(diethoxy-phosphoryl-acétyl]-amino}-3-méthyl-butyrylamino) acétate d'éthyle.

Les composés selon l'invention peuvent être aisément préparés par l'homme du métier sur base de ses connaissances générales. On peut notamment faire réagir ensemble un acide carboxylique, un aldéhyde, un composé aminé et un isonitrile, selon la réaction de Ugi.
Bien entendu, lors de la synthèse des composés selon l'invention, et en fonction de la nature des différents radicaux présents sur les composés de départ, l'homme du métier pourra veiller à protéger certains substituants pour que ceux-ci n'interviennent pas dans la suite des réactions.

La quantité de composé à utiliser dans les compositions selon l'invention peut être aisément déterminée par l'homme du métier, en fonction de la nature du composé utilisé, de la personne à traiter et/ou de l'effet recherché. D'une manière générale, cette quantité peut être comprise entre 0,00001 et 20% en poids par rapport au poids total de la composition, notamment entre 0,001 et 10% en poids, et de préférence entre 0,05 et 5% en poids, encore mieux entre 0,1 et 2% en poids, et préférentiellement entre 0,5 et 1 % en poids.

Les composés de formule (I) peuvent notamment être employés, seul ou en mélange, dans une composition qui comprend un milieu physiologiquement acceptable, notamment dans une composition cosmétique ou pharmaceutique qui comprend donc par ailleurs un milieu cosmétiquement ou pharmaceutiquement acceptable.
Le milieu physiologiquement acceptable dans lequel les composés selon l'invention peuvent être employés, ainsi que ses constituants, leur quantité, la forme galénique de la composition et son mode de préparation, peuvent être choisis par l'homme du métier sur la base de ses connaissances générales en fonction du type de composition recherchée.
D'une manière générale, ce milieu peut être anhydre ou aqueux. Il peut ainsi comprendre une phase aqueuse et/ou une phase grasse.

Pour une application sur la peau, la composition peut avoir la forme notamment de solution aqueuse ou huileuse; de dispersion du type lotion ou sérum; d'émulsions de consistance liquide ou semi-liquide du type lait obtenues par dispersion d'une phase grasse dans une phase aqueuse (H/E) ou inversement (E/H); de suspensions ou émulsions de consistance molle du type crème ou gel aqueux ou anhydres; de microcapsules ou microparticules; de dispersions vésiculaires de type ionique et/ou non ionique.
Pour une application sur les cheveux, la composition peut être sous forme de solutions aqueuses, alcooliques ou hydroalcooliques; sous forme de crèmes, de gels, d'émulsions, de mousses; sous forme de compositions pour aérosol comprenant également un agent propulseur sous pression.

Lorsque la composition se présente sous forme aqueuse, notamment sous forme de dispersion, d'émulsion ou de solution aqueuse, elle peut comprendre une phase aqueuse, qui peut comprendre de l'eau, une eau florale et/ou une eau minérale.
Ladite phase aqueuse peut comprendre en outre des alcools tels que des monoalcools en C₁-C₆ et/ou des polyols tels que le glycérol, le butylèneglycol, l'isoprène glycol, le propylèneglycol, le polyéthylèneglycol.

Lorsque la composition selon l'invention se présente sous la forme d'une émulsion, elle peut éventuellement comprendre en outre un tensioactif, de préférence en une quantité de 0,01 à 30% en poids par rapport au poids total de la composition. La composition selon l'invention peut également comprendre au moins un co-émulsionnant qui peut être choisi parmi le monostéarate de sorbitan oxyéthyléné, des alcools gras tels que l'alcool stéarylique ou l'alcool cétylique, ou des esters d'acides gras et de polyols tels que le stéarate de glycéryle.

La composition selon l'invention peut également comprendre une phase grasse, notamment constituée de corps gras liquides à 25°C, tels que des huiles d'origine animale, végétale, minérale ou synthétique, volatiles ou non; de corps gras solides à 25°C tels que des cires d'origine animale, végétale, minérale ou synthétique; de corps gras pâteux; de gommes; de leurs mélanges.
Les huiles volatiles sont généralement des huiles ayant, à 25°C, une tension de vapeur saturante au moins égale à 0,5 millibar (soit 50 Pa).
Parmi les constituants de la phase grasse, on peut citer :
- les silicones volatiles cycliques ayant de 3 à 8 atomes de silicium, de préférence de 4 à 6.
- les cyclocopolymères du type diméthylsiloxane/méthylalkylsiloxane,
- les silicones volatiles linéaires ayant de 2 à 9 atomes de silicium.
- les huiles volatiles hydrocarbonées, telles que les isoparaffines et notamment l'isododécane et des huiles fluorées.
- les polyalkyl(C₁-C₂₀) siloxanes et notamment ceux à groupements terminaux triméthylsilyle, parmi lesquels on peut citer les polydiméthylsiloxanes linéaires et les alkylméthylpolysiloxanes tels que la cétyldiméthicone (nom CTFA),
- les silicones modifiées par des groupements aliphatiques et/ou aromatiques, éventuellement fluorés, ou par des groupements fonctionnels tels que des groupements hydroxyles, thiols et/ou amines.
- les huiles de silicone phénylées,
- les huiles d'origine animale, végétale ou minérale, et notamment les huiles animales ou végétales formées par des esters d'acide gras et de polyols, en particulier les triglycérides liquides, par exemple les huiles de tournesol, de maïs, de soja, de courge, de pépins de raisin, de sésame, de noisette, d'abricot, d'amandes ou d'avocat; les huiles de poisson, le tricaprocaprylate de glycérol, ou les huiles végétales ou animales de formule R1COOR₂ dans laquelle R₁ représente le reste d'un acide gras supérieur comportant de 7 à 19 atomes de carbone et R₂ représente une chaîne hydrocarbonée ramifiée contenant de 3 à 20 atomes de carbone, par exemple, l'huile de Purcellin; l'huile de paraffine, de vaseline, le perhydrosqualène, l'huile de germes de blé, de calophyllum, de sésame, de macadamia, de pépins de raisin, de colza, de coprah, d'arachide, de palme, de ricin, de jojoba, d'olive ou de germes de céréales; des esters d'acides gras; des alcools; des acétylglycérides; des octanoates, décanoates ou ricinoléates d'alcools ou de polyalcools; des triglycérides d'acides gras; des glycérides;
- les huiles fluorées et perfluorées.
- les gommes de silicones;
- les cires d'origine animale, végétale, minérale ou synthétique telles que les cires microcristallines, la paraffine, le pétrolatum, la vaseline, l'ozokérite, la cire de montan; la cire d'abeilles, la lanoline et ses dérivés; les cires de Candellila, d'Ouricury, de Carnauba, du Japon, le beurre de cacao, les cires de fibres de lièges ou de canne à sucre; les huiles hydrogénées concrètes à 25°C, les ozokérites, les esters gras et les glycérides concrets à 25°C; les cires de polyéthylène et les cires obtenues par synthèse de Fischer-Tropsch; des huiles hydrogénées concrètes à 25°C; des lanolines; des esters gras concrets à 25°C; les cires de silicone; les cires fluorées.

De façon connue, la composition selon l'invention peut comprendre les adjuvants habituels dans le domaine considéré, tels que les gélifiants hydrophiles ou lipophiles, les additifs hydrophiles ou lipophiles, les actifs notamment cosmétiques
ou pharmaceutiques hydrophiles ou lipophiles, les conservateurs, les antioxydants, les solvants, les parfums, les charges, les pigments, les nacres, les filtres UV, les absorbeurs d'odeur et les colorants. Ces adjuvants, selon leur nature, peuvent être introduits dans la phase grasse, dans la phase aqueuse et/ou dans des sphérules lipidiques.
La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Les compositions cosmétiques ou pharmaceutiques selon l'invention peuvent notamment se présenter sous la forme d'une composition destinée au soin et/ou au traitement des zones ulcérées ou ayant subi un stress ou microstress cutané, notamment généré par une exposition aux UV et/ou la mise en contact avec un produit irritant.

Ainsi, les compositions selon l'invention peuvent notamment se présenter sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées; une composition démaquillante; un lait pour le corps, notamment hydratant éventuellement après-soleil;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux; un shampooing antiparasitaire;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

Les compositions selon l'invention trouvent une application préférée comme composition de soin de la peau du visage, de type anti-rides ou anti-age, et comme composition de protection solaire ou après-soleil.

La présente invention a également pour objet un procédé de traitement cosmétique de la peau du corps ou du visage, y compris le cuir chevelu, dans lequel on applique sur la peau une composition cosmétique comprenant une quantité efficace d'au moins un composé de formule (I), à laisser celle-ci en contact puis éventuellement à rincer.
Le procédé de traitement cosmétique de l'invention peut être mis en oeuvre notamment en appliquant les compositions cosmétiques telles que définies ci-dessus, selon la technique d'utilisation habituelle de ces compositions. Par exemple : application de crèmes, de gels, de sérums, de lotions, de laits de démaquillage ou de compositions anti-solaires sur la peau ou sur les cheveux secs; application d'une lotion pour cuir chevelu sur cheveux mouillés; application de dentifrice sur les gencives.

L'invention est illustrée plus en détail dans les exemples suivants.

### Exemple 1

Préparation du {2-[acétyt-(3-triftuorométhyl-phényl)-amino]-3-méthyl-butyrylamino} acétate d'éthyle de formule :

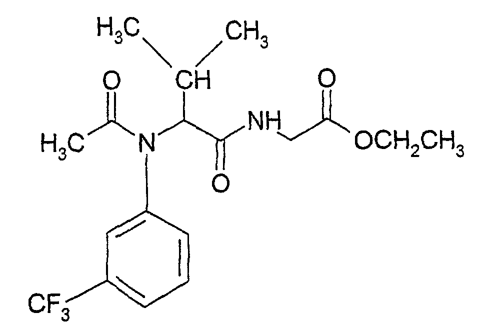

On mélange 0,63 ml d'isobutyraldéhyde et 1 ml de trifluorométhylaniline (1,15 eq) dans 15 ml de méthanol, sous agitation. On laisse réagir 15 minutes à 20°C, puis on ajoute 0,46 ml d'acide acétique (1,15 eq) et on laisse réagir 10 minutes à 20°C. On ajoute alors 0,8 ml d'isocyanoacétate d'éthyle à 95% (1 eq) et on laisse réagir 48 heures à 20°C.
On concentre le milieu réactionnel au rotovapor et on purifie le résidu sur colonne de silice (éluant : heptane : 3 / acétate d'éthyle : 7; Rf = 0,5).
On obtient 2,45 g de composé sous forme de solide cireux, d'où un rendement de 91%.
RMN ¹H (200 MHz; CDCI3) δ ppm : 0,9 (6H;q), 1,3 (3H;t), 1,8 (3H;s), 2,3 (1H;m), 4,0 (2H,q), 4,2 (2H;q), 4,4 (2H;d), 7,3 (1 H;t), 7,5 (4H;m)

### Exemple 2

Préparation de l'acide {2-[acétyl-(3-trifluorométhyl-phényl)-amino]-3-méthylbutyrylamino} acétique de formule :

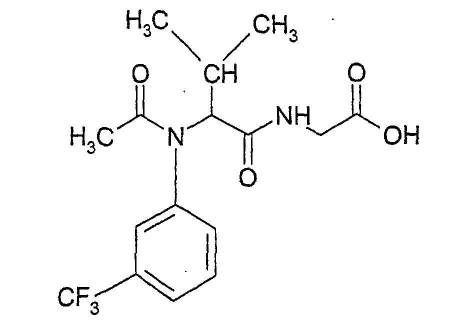

On solubilise 2 g de composé préparé à l'exemple 1 dans 30 ml d'acétone. On ajoute 30 ml de soude 2N et on laisse réagir pendant 6 heures à 20°C. On concentre le milieu réactionnel au rotovapor. On acidifie la phase aqueuse résiduelle à pH 2 par ajout d'HCl concentré puis on extrait par CH2Cl2.
La phase organique est concentrée à sec après séchage sur sulfate de sodium.
On obtient un résidu qui est solubilisé par un mélange eau basique à 10% d'éthanol, puis de nouveau acidifié par HCl concentré à pH 2. On extrait une nouvelle fois par Ch2Cl2 et on sèche la phase organique sur sulfate de sodium, on la filtre et on la concentre à sec sous vide au rotovapor.

On obtient 1,3 g de composé sous forme d'un solide légèrement marron clair, d'où un rendement de 70%.

RMN ¹H (200 MHz; DMSO) δ ppm : 0,9 (6H;q), 3,7 (2H;m), 1,8 (4H;m), 4,8 (2H;d), 7,6 (4H,m),8,4 (1 H;t), 12,5 (1 H;s)

### Exemple 3

On a déterminé in vitro l'activité anti-élastasique de composés selon l'invention, vis-à-vis de l'élastase leucocytaire humaine (ELH).

### Le test est effectué de la manière suivante :

On laisse incuber, à 37°C, pendant 60 minutes, un substrat Me-OSAAPV-p-NA (Méthyl-O-succinate alanine alanine proline valine-p-nitroanilide) sur lequel est appliqué de l'ELH (40 milli-unités par ml) et 0,1 % du composé à tester.

On détermine ensuite par spectrophotométrie le % d'inhibition de l'activité élastase témoin.

Les composés testés sont les suivants :
Composé A : acide {2-[acétyl-(3-trifluorométhyl-phényl)-amino]-3-méthyl-butyrylamino} acétique
Composé B : (2-{benzyl-[(diethoxy-phosphoryl)-acétyl]-amino}-3-méthyl-butyrylamino) acétate d'éthyle
Composé C : acide [2-(acétyl-benzyl-amino)-3-méthyl-butyrylamino] acétique
Composé D : [2-(acétyl-benzyl-amino)-3-méthyl-butyrylamino] acétate d'éthyle

On obtient les résultats suivants :

| Composé (concentration : 0,1%) | % d'inhibition de l'activité élastase témoin |
|---|---|
| Composé A | 67% |
| Composé B | 17% |
| Composé C | 20% |
| Composé D | 13% |

On détermine de la même manière le % d'inhibition de l'activité élastase témoin pour le composé A, à différentes concentrations.

On obtient les résultas suivants :

| Concentration du composé A | % d'inhibition de l'activité élastase témoin |
|---|---|
| 0,01 % | 53% |
| 0,05% | 50% |
| 0,1% | 68% |
| 0,2% | 68% |

Le composé A provoque donc une forte inhibition de l'activité élastase, même en une quantité faible.

### Exemple 4

On a évalué l'activité ex vivo du composé de l'exemple 2 sur des peaux humaines en survie traitées par de l'élastase leucocytaire humaine (ELH).

Le test est effectué de la manière suivante :
Des coupes fraîches de peaux humaines, provenant de 2 donneurs différents, sont traitées pendant 2 heures, à 20°C, par 20 µl de solution tampon (pH 7,4) comprenant éventuellement 10 µg/ml d'ELH et éventuellement 0,1% du composé à tester, éventuellement préalablement mis en solution dans l'éthanol.
Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques | |
|---|---|---|
| | Peau 1 | Peau 2 |
| Témoin (peau non traitée) | 12,7% | 15,25% |
| Peau traitée avec ELH | 4,85% | 6,85% |
| Peau traitée avec ELH + composé de l'exemple 2 | 13,95% | 11,85% |

On constate donc que le composé selon l'invention génère une protection significative des peaux vis-à-vis de la destruction des fibres élastiques induite par l'élastase.

### Exemple 5

On a évalué l'activité ex vivo du composé de l'exemple 2 sur des peaux humaines en survie traitées par de l'élastase leucocytaire humaine (ELH).

Le test est effectué de la manière suivante :
Des fragments de peau humaine normale provenant de trois donneurs différents sont déposées dans des inserts positionnés dans des puits de culture. On ajoute
du milieu de culture supplémenté en antibiotiques dans le fond des puits. Un passage s'effectue par diffusion lente entre les deux compartiments par l'intermédiaire d'une membrane poreuse (taille des pores : 12 µm).
Le milieu de culture est renouvelé tous les trois jours.

On ajoute sur les fragments de peau éventuellement 0,5 µg d'ELH par ml de milieu de culture.
On ajoute également, tous les deux jours, 5 µl du composé à tester, préalablement mis en solution à 0,2% en poids dans l'éthanol.
Les peaux sont maintenues en survie pendant 10 jours à 37°C.
Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | % de surface occupée par les fibres élastiques |
|---|---|
| Témoin (peau non traitée) | 7,4% |
| Peau traitée avec ELH | 5,1% |
| Peau traitée avec ELH + composé de l'exemple 2 | 7,1% |

On constate donc que le composé selon l'invention génère une protection significative des peaux vis-à-vis de la destruction des fibres élastiques induite par l'élastase.

### Exemple 6

On a évalué l'activité du composé de l'exemple 2 sur des peaux humaines en survie irradiées par des UVA (8 J/cm²).

### Le test est effectué de la manière suivante :

Des fragments de peau humaine normale provenant de quatre donneurs différents sont déposées dans des inserts positionnés dans des puits de culture. On ajoute du milieu de culture supplémenté en antibiotiques dans le fond des puits. Un passage s'effectue par diffusion lente entre les deux compartiments par l'intermédiaire d'une membrane poreuse (taille des pores : 12 µm).
Le milieu de culture est renouvelé tous les trois jours.

On ajoute sur les fragments de peau, tous les deux jours, 5 µl du composé à tester, préalablement mis en solution à 0,2% dans l'éthanol.
Les peaux sont maintenues en survie pendant 7 jours à 37°C.
Les peaux sont irradiées une seule fois à 8 J/cm² (lampe Vilbert-Lourmat RMX-3W).
Les fibres élastiques sont colorées en bleu à la (+) catéchine et quantifiées morphométriquement par analyse d'image assistée par ordinateur. Le pourcentage de surface de derme moyen occupé par les fibres élastiques est ainsi évalué.

On obtient les résultats suivants :

| | Analyse morphométrique des fibres élastiques (derme superficiel) | Analyse morphométrique du collagène (derme superficiel) |
|---|---|---|
| Peau non traitée | 6,75% | 87% |
| Peau traitée par des UVA (8 J/cm²) | 3,9% | 81 % |
| Peau traitée par des UVA (8 J/cm²) + composé | 6,8% | 92% |

On constate que le composé selon l'invention a bien une activité vis-à-vis de la dégradation des fibres élastiques dans le derme superficiel de peaux irradiées par des UVA.
Ce composé présente également un effet adéquat sur la protection du collagène.

### Exemple 7 : composition pour application topique

On prépare l'émulsion suivante de façon classique (% en poids) :
- composé de l'Exemple 1 1 %
- isostéarate de propylène glycol 13 %
- polyéthylène glycol (8 OE) 5 %
- propylène glycol 3 %
- pentylène glycol 3 %
- stéarate de glycéryle et stéarate de polyéthylène glycol (100 OE) 5 %
- mono-stéarate de sorbitane oxyéthyléné (20 OE) 0,5 %
- alcool cétylique oxyéthyléné (20 OE) oxypropyléné (5 OP) 1 %
- gélifiants 0,5 %
- benzoates d'alkyle en C₁₂₋₁₅ 4 %
- éthanol 3 %
- hydroxyde de sodium 0,12 %
- conservateurs qs
- eau qsp 100 %

### Exemple 8 : crème de soin du visage

On prépare l'émulsion huile-dans-eau suivante de façon classique (% en poids) :
- Composé de l'exemple 2 1 %
- Stearate de glycérol 2 %
- Polysorbate 60 (Tween 60® vendu par la société ICI) 1 %
- Acide stéarique 1,4 %
- Triéthanolamine 0,7 %
- Carbomer 0,4 %
- Fraction liquide du beurre de karité 12 %
- Perhydrosqualène 12 %
- Antioxydant qs
- Parfum qs
- Conservateur qs
- Eau qsp 100 %

### Exemple 9 : lait pour le visage

On prépare le lait suivant de façon classique (% en poids) :
- Huile de vaseline 7 %
- Composé de l'exemple 2 1 %
- Monostéarate de glycéryle, stéarate de polyéthylène glycol (100 OE) 3 %
- Polymère carboxyvinylique 0,4 %
- Alcool stéarylique 0,7 %
- Protéines de soja 3 %
- NaOH 0,4%
- Conservateur qs
- Eau qsp 100 %

### Exemple 10 : lotion capillaire

On prépare la lotion suivante de façon classique (% en poids) :
- composé de l'Exemple 1 1 %
- propylène glycol 23 %
- éthanol 55 %
- eau qsp 100 %

On peut appliquer cette lotion sur le scalp des individus alopéciques, pour prévenir les effets des UV, avant et/ou après exposition au soleil.

### Exemple 11 : lotion antichute

On prépare la lotion suivante de façon classique (% en poids) :
- composé de l'Exemple 2 1 %
- propylène glycol 23 %
- éthanol 55 %
- Aminexil 1,5 %
- eau qsp 100 %

On peut appliquer cette lotion antichute sur le scalp des individus alopéciques.

## Revendications

1. Composé de formule (1) : 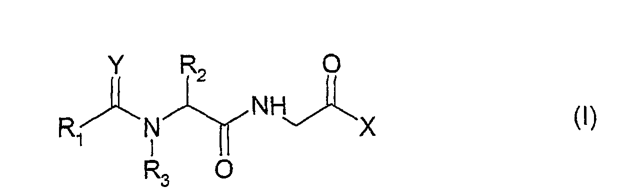 dans laquelle:
- le radical Y représente O ou S,
- le radical R1 représente :
- (i) un atome d'hydrogène;
- (ii) un radical hydrocarboné, linéaire, ramifié ou cyclique, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR; -P(O)-(OR)₂; -SO₂-OR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR";
-NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- (iii) un radical choisi parmi les radicaux -OR; -NH₂; -NHR; -NRR'; -NH-COR; -COOR; -COR ;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR";
(NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R2 représente un radical hydrocarboné, linéaire, ramifié ou cyclique, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène); -CN; -COOR; -COR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical R3 représente un radical choisi parmi ceux de formule (II) ou (III) :
(II) -A-C₆H_{(5-y)}-B_{y}
(III) -C₆H_{(5-y')}-B_{y'}
dans lesquelles:
- y est un entier compris entre 0 et 5 inclus, et y' est un entier compris entre 1 et 5 inclus;
- A est un radical divalent hydrocarboné, linéaire ou ramifié, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- B représente au moins un groupement, identique ou différent, choisi parmi -OH; -OR; -O-COR; -SH; -SR; -S-COR; -NH₂; -NHR; -NRR'; -NH-COR; -Halogène; -CN; -COOR; -COR; -NO₂; -SO₂-OR, ou représente un radical hydrocarboné, linéaire ou ramifié, saturé ou insaturé, ayant 1 à 18 atomes de carbone,
éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; -NO₂; -SO₂-OR;
avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné,
lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
- le radical X représente un radical choisi parmi -OH, -OR₄, -NH₂, -NHR₄, -NR₄R₅ , -SR₄, -COOR₄; -COR₄;
avec R₄ et R₅ représentant, indépendamment l'un de l'autre, un radical hydrocarboné, linéaire, cyclique ou ramifié, saturé ou insaturé, ayant 1 à 6 atomes de carbone, éventuellement substitué par 1 à 5 groupements, identiques ou différents, choisis parmi -OH; -OR; -O-COR; -SH; -SR; -S-COR; -NH₂; -NHR; -NRR'; -NH-COR; -Hal (halogène, voire perhalogène); -CN; -COOR; -COR; avec R et R' représentant, indépendamment l'un de l'autre, un radical hydrocarboné, linéaire, ramifié ou cyclique, saturé ou insaturé, ayant 1 à 6 atomes de carbone, éventuellement halogéné, voire perhalogéné; lesdits radicaux R et R' pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
lesdits radicaux R₄ et R₅ pouvant former ensemble avec N un cycle carboné à 5 ou 6 chaînons pouvant comprendre en outre au moins un hétéroatome choisi parmi O, N et/ou S dans le cycle, et/ou pouvant être substitué par 1 à 5 groupements, identiques ou différents, choisis parmi -OH; -OR"; -O-COR"; -SH; -SR"; -S-COR"; -NH₂; -NHR"; -NH-COR"; -Hal (halogène); -CN; -COOR"; -COR"; avec R" représentant un radical hydrocarboné, linéaire, ramifié ou cyclique, saturé ou insaturé, ayant 1 à 6 atomes de carbone, éventuellement halogéné, voire perhalogéné;
ses sels d'acide minéral ou organique, ses isomères optiques, sous forme isolée ou en mélange racémique,
à l'exception des dérivés suivants :
- *N*-Acétylglycyl-*N*-(benzyl)-DL-valylglycine *tert-*butyl ester ;
- *N*-Acétylglycyl-*N*-(2-nitrobenzyl)-DL-valylglycine *tert*-butyl ester ;
- *N*-Acétylglycyl-*N*-(2,4-diméthyloxybenzyl)-DL-valylglycine *tert*-butyl ester.

2. Composé selon la revendication 1 dans lequel:
- le radical Y représente l'oxygène, et/ou
- le radical R1 représente l'hydrogène ou un radical hydrocarboné, linéaire ou ramifié, saturé ou insaturé, ayant 1 à 12, et notamment 1, 2, 3, 4, 5 ou 6, atomes de carbone, éventuellement substitué, et/ou
- les substituants de R1 sont choisis parmi -OH, -OR et/ou -P(O)-(OR)₂ avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R2 représente un radical hydrocarboné, linéaire, ramifié ou cyclique, saturé ou insaturé, ayant 1 à 12, notamment 1, 2, 3, 4, 5 ou 6, atomes de carbone, éventuellement substitué; et/ou
- les substituants de R2 sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné; et/ou
- le radical R3 représente un radical de formule -C₆H(_{5-y}2,)-B_{y'} pour lequel y' = 1, 2 ou 3; ou un radical de formule -A-C₆H(_{5-y})-B_{y} pour lequel y = 0, 1 ou 2; et/ou
- le radical A de R3 est un radical divalent hydrocarboné, linéaire ou ramifié, saturé ou insaturé, ayant 1 à 12 atomes de carbone, éventuellement substitué; et/ou
- le radical B de R3 représente au moins un groupement -OR; -NHR; -CN; -COOR;
- COR ou représente un radical hydrocarboné, linéaire ou ramifié, saturé ou insaturé, ayant 1 à 12 atomes de carbone, éventuellement substitué; et/ou
- les substituants de A et/ou de B sont choisis parmi -Hat (halogène, voire perhalogène); -CN; -COOR; -NO₂; -SO₂-OR; avec R représentant un radical hydrocarboné, linéaire, ramifié ou cyclique, saturé ou insaturé, ayant 1 à 6 atomes de carbone, éventuellement halogéné, voire perhalogéné; et/ou
- le radical X représente un radical choisi parmi -OH ou -OR₄ avec R₄ représentant un radical hydrocarboné, linéaire, cyclique ou ramifié, saturé ou insaturé, ayant 1 à 6 atomes de carbone, éventuellement substitué; et/ou
- les substituants de R4 de X sont choisis parmi -OH et -OR avec R représentant un radical hydrocarboné, linéaire, ramifié ou cyclique, saturé ou insaturé, ayant 1 à 6 atomes de carbone, éventuellement halogéné, voire perhalogéné.

3. Composé selon l'une des revendications précédentes, dans lequel :
- le radical R1 représente un radical méthyle, éthyle, propyle ou isopropyle, éventuellement substitué par un groupement -OH ou -P(O)-(OR)₂ avec R représentant méthyle, éthyle, propyle ou isopropyle; et/ou
- le radical R2 représente un radical méthyle, éthyle, propyle, isopropyle, n-butyle, ter-butyle ou isobutyle; et/ou
- le radical R3 représente un groupement choisi parmi l'une des formules suivantes : 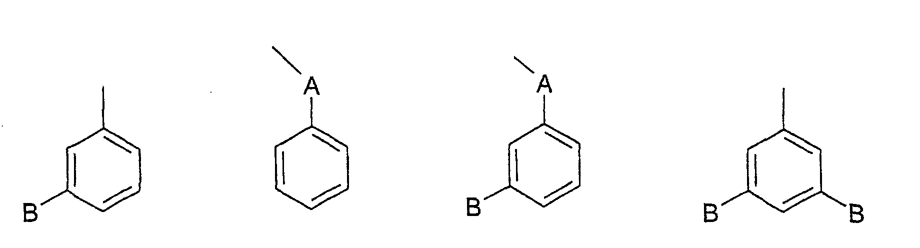 dans lesquelles le radical divalent A est un méthylène, un éthylène, un propylène et/ou le radical B représente au moins un groupement -OR; -NHR; -CN; -COOR; -COR pour lesquels R désigne un radical méthyle, éthyle, propyle ou isopropyle, ou représente un radical hydrocarboné choisi parmi un radical méthyle, éthyle, propyle ou isopropyle, substitué par un ou plusieurs halogènes, notamment chlore, brome, iode ou fluor, et préférentiellement totalement halogéné (perhalogéné), tel que perfluoré, notamment le radical perfluorométhyle (-CF3).
- le radical X représente un radical choisi parmi -OH, -OCH₃, -OC₂H₅, -O-C₃H₇ ou - OC₄H₉.

4. Composé selon l'une des revendications précédentes, de nom chimique :
- l'acide {2-[acétyl-(3-trifluorométhyl-phényl)-amino]-3-méthyt-butyry)amino} acétique,
- le {2-[acétyl-(3-trifluorométhyl-phényl)-amino]-3-méthyl-butyrylamino} acétate d'éthyle,
- l'acide {2-(acétyl-benzyl-amino)-3-méthyl-butyrylamino] acétique,
- le [2-(acétyl-benzyl-amino)-3-méthyl-butyrylamino] acétate d'éthyle,
- le (2-{benzyl-[{diethoxy-phosphoryl)-acétyl]-amino}-3-méthyl-butyrylamino) acétate d'éthyle.

5. Composition comprenant, dans un milieu physiologiquement acceptable, au moins un composé de formule (I) tel que défini dans l'une des revendications 1 à 4.

6. Composition selon la revendication 5, dans laquelle le composé de formule (1) est présent en une quantité comprise entre 0,00001 et 20% en poids par rapport au poids total de la composition, notamment entre 0,001 et 10% en poids, et de préférence entre 0,05 et 5% en poids, encore mieux entre 0,1 et 2% en poids, et préférentiellement entre 0,5 et 1 % en poids.

7. Composition selon l'une des revendications 5 à 6, se présentant sous la forme d'une composition cosmétique ou pharmaceutique destinée au soin et/ou au traitement des zones ulcérées ou ayant subi un stress ou microstress cutané, notamment généré par une exposition aux UV et/ou la mise en contact avec un produit irritant.

8. Composition selon l'une des revendications 5 à 6, se présentant sous la forme :
- d'un produit de soin, de traitement, de nettoyage ou de protection, de la peau du visage ou du corps y compris le cuir chevelu, tel qu'une composition de soin (de jour, de nuit, hydratante) du visage ou du corps; une composition anti-rides ou anti-age pour le visage; une composition matifiante pour le visage; une composition pour les peaux irritées;
- d'une composition de protection solaire, de bronzage artificiel (autobronzant) ou de soin après-soleil;
- d'une composition capillaire, et notamment une crème ou un gel protecteur solaire; une composition de soin du cuir chevelu, notamment anti-chute ou repousse des cheveux;
- d'un produit de maquillage de la peau du visage, du corps ou des lèvres, tel qu'un fond de teint, une crème teintée, un fard à joues ou à paupières, une poudre libre ou compacte, un stick anti-cernes, un stick camouflant, un rouge à lèvres, un soin des lèvres;
- d'un produit d'hygiène buccale tel qu'une pâte dentifrice ou une lotion de rinçage buccal.

9. Composition selon l'une des revendications 5 à 8, se présentant sous la forme d'une composition de soin de la peau du visage, de type anti-rides ou anti-age, ou d'une composition de protection solaire ou après-soleil.

10. Utilisation d'au moins un composé de formule (I) tel que défini dans l'une des revendications 1 à 4, ou d'une composition le comprenant telle-que définie dans l'une des revendications 5 à 9, pour la préparation d'un médicament destiné à traiter, de manière préventive ou curative, les signes du vieillissement de la peau du corps ou du visage, qu'il soit chronobiologique ou photo-induit, et notamment le vieillissement généré par une diminution de l'élasticité de la peau et/ou par une dégradation du collagène dans la structure des tissus.

11. Utilisation d'au moins un composé de formule (I) tel que défini dans l'une des revendications 1 à 4, ou d'une composition le comprenant telle que définie dans l'une des revendications 5 à 9, pour la préparation d'un médicament destiné à traiter de manière preventive ou curative, les rides et/ou ridules, la peau flétrie, le manque d'élasticité et/ou de tonus de la peau, l'amincissement du derme, la dégradation des fibres de collagène, la peau molle, la peau amincie; les dégradations internes de la peau consécutives à une exposition aux rayonnements ultra-violets.

12. Utilisation d'au moins un composé de formule (I) tel que défini dans l'une des revendications 1 à 4, ou d'une composition le comprenant telle que définie dans l'une des revendications 5 à 9, pour la préparation d'un médicament destiné à inhiber l'activité des élastases et/ou pour limiter et/ou combattre la dégradation des fibres élastiques.

13. Procédé de traitement cosmétique de la peau du corps ou du visage, y compris le cuir chevelu, dans lequel on applique sur la peau une composition cosmétique telle que définie dans l'une des revendications 5 à 9.

## Patentansprüche

1. Verbindung der Formel (I): 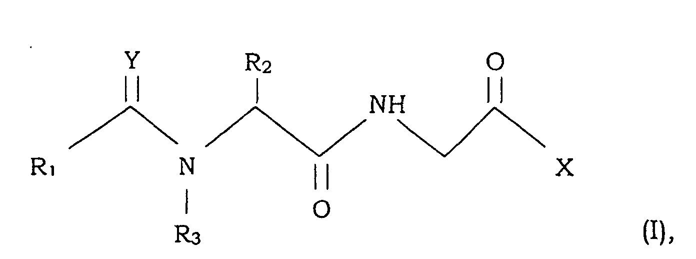 worin bedeuten:
- die Gruppe Y O oder S,
- die Gruppe R₁:
- (i) ein Wasserstoffatom,
- (ii) eine geradkettige, verzweigte oder cyclische, gesättigte oder ungesättigte Kohlenwasserstoffgruppe mit 1 bis 18 Kohlenstoffatomen,
die gegebenenfalls mit 1 bis 5 Gruppen substituiert sein kann, die gleich oder verschieden sein können und die unter den folgenden Gruppen ausgewählt sind: -OH; -OR; -O-COR; -SH; -SR; -S-COR; -NH₂; -NHR; -NRR'; -NH-COR; -Hal (Halogen); -CN; -COOR; -COR; -P(O)-(OR)₂; -SO₂-OR;
wobei R und R' unabhängig voneinander einer geradkettige, verzweigte oder cyclische, gesättigte oder ungesättigte Kohlenwasserstoffgruppe mit 1 bis 6 Kohlenstoffatomen bedeuten, die gegebenenfalls halogeniert oder sogar perhalogeniert sein kann,
wobei die Gruppen R und R' gemeinsam mit N einen 5-oder 6-gliedrigen Kohlenstoffring bilden können, der im Ring auch mindestens ein Heteroatom enthalten kann, das unter O, N und/oder S ausgewählt ist, und/oder mit 1 bis 5 gleichen oder verschiedenen Gruppen substituiert sein kann, die unter den folgenden Verbindungen ausgewählt sind: -OH; -OR"; -O-COR"; -SH; -SR"; -S-COR"; NH₂; -NHR"; -NH-COR"; -Hal (Halogen); -CN; -COOR"; -COR", wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert sein kann;
- (iii) eine Gruppe, die unter den Gruppen -OR; -NH₂; -NHR;
- NRR'; -NH-COR; -COOR; -COR ausgewählt ist;
wobei R und R' unabhängig voneinander eine geradkettige, verzweigte oder cyclische, gesättigte oder ungesättigte Kohlenwasserstoffgruppe mit 1 bis 6 Kohlenstoffatomen bedeuten, die gegebenenfalls halogeniert oder sogar perhalogeniert ist,
wobei die Gruppen R und R' gemeinsam mit N einen 5-oder 6-gliedrigen Kohlenstoffring bilden können, der außerdem im Ring mindestens ein Heteroatom enthalten kann, das unter O, N und/oder S ausgewählt ist, und/oder mit 1 bis 5 gleichen oder verschiedenen Gruppen substituiert sein kann, die unter den folgenden Gruppen ausgewählt sind: -OH; -OR"; -O-COR"; -SH; -SR"; -S-COR"; -NH₂; -NHR"; -NH-COR"; -Hal (Halogen); -CN; -COOR"; -COR" wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist;
- die Gruppe R₂ eine geradkettige, verzweigte oder cyclische, gesättigte oder ungesättigte Kohlenwasserstoffgruppe mit 1 bis 18 Kohlenstoffatomen,
die gegebenenfalls mit 1 bis 5 gleichen oder verschiedenen Gruppen substituiert ist, die unter den Gruppen -OH; -OR; -O-COR; -SH; -SR; -S-COR; -NH₂; -NHR; -NRR'; -NH-COR; -Hal (Halogen); -CN; -COOR; -COR ausgewählt ist,
wobei R und R' unabhängig voneinander eine geradkettige, verzweigte oder cyclische, gesättigte oder ungesättigte Kohlenwasserstoffgruppe mit 1 bis 6 Kohlenstoffatomen bedeuten, die gegebenenfalls halogeniert oder sogar perhalogeniert ist,
wobei die Gruppen R und R' gemeinsam mit N einen 5- oder 6-gliedrigen Kohlenstoffring bilden können, der auch mindestens ein Heteroatom im Ring enthalten kann, das unter O, N und/oder S ausgewählt ist, und/oder mit 1 bis 5 identischen oder voneinander verschiedenen Gruppen substituiert sein kann, die unter -OH; -OR"; -O-COR"; -SH; -SR"; -S-COR";
-NH₂; -NHR"; -NH-COR"; -Hal (Halogen); -CN; -COOR"; -COR" ausgewählt sind, wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist;
- die Gruppe R₃ eine Gruppe, die unter den Gruppen der Formeln (II) oder (III) ausgewählt ist:
(II) -A-C₆H_{(5-y)}-B_{y}
(III) -C₆H(_{5-y'})-B_{y'}
worin bedeuten:
- y 0 oder eine ganze Zahl von 1 bis 5, wobei die Grenzen eingeschlossen sind, und y' eine ganze Zahl von 1 bis 5, wobei die Grenzen eingeschlossen sind;
- A eine geradkettige oder verzweigte, gesättigte oder ungesättigte zweiwertige Kohlenwasserstoffgruppe mit 1 bis 18 Kohlenstoffatomen,
die gegebenenfalls mit 1 bis 5 gleichen oder verschiedenen Gruppen substituiert sein kann, die unter -OH; -OR; -O-COR; -SH; -SR; -S-COR; -NH₂; -NHR; -NRR'; -NH-COR; -Hal (Halogen oder sogar Perhalogen); -CN; -COOR; -COR; -NO₂; -SO₂-OR ausgewählt sind,
wobei R und R' unabhängig voneinander eine geradkettige, verzweigte oder cyclische, gesättigte oder ungesättigte Kohlenwasserstoffgruppe mit 1 bis 6 Kohlenstoffatomen bedeuten, die gegebenenfalls halogeniert oder sogar perhalogeniert ist,
wobei die Gruppen R und R' gemeinsam mit N einen 5- oder 6-gliedrigen Kohlenstoffring bilden können, der ferner mindestens ein Heteroatom im Ring enthalten kann, das unter O, N und/oder S ausgewählt ist, und/oder mit 1 bis 5 gleichen oder verschiedenen Gruppen substituiert sein kann, die unter -OH; -OR"; -O-COR"; -SH; -SR"; -S-COR"; NH₂; - NHR"; -NH-COR"; -Hal (Halogen); -CN; -COOR"; -COR" ausgewählt sind;
wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist;
- B mindestens eine Gruppe die ausgewählt ist unter: -OH; -OR;
-O-COR; -SH; -SR; -S-COR; -NH₂; -NHR; -NRR'; -NH-COR;
-Halogen; -CN; -COOR; -COR; -NO₂; -SO₂-OR, wobei diese Gruppen gleich oder verschieden sein können, oder B bedeutet eine geradkettige oder verzweigte, gesättigte oder ungesättigte Kohlenwasserstoffgruppe mit 1 bis 18 Kohlenstoffatomen, die gegebenenfalls mit 1 bis 5 gleichen oder verschiedenen Gruppen substituiert sein kann, die unter -OH; -OR; -O-COR; -SH;
-SR; -S-COR; -NH₂; -NHR; -NRR'; -NH-COR; -Hal (Halogen oder sogar Perhalogen); -CN; -COOR; -COR; -NO₂; -SO₂-OR ausgewählt ist,
wobei R und R' unabhängig voneinander eine geradkettige, verzweigte oder cyclische, gesättigte oder ungesättigte Kohlenwasserstoffgruppe mit 1 bis 6 Kohlenstoffatomen bedeuten, die gegebenenfalls halogeniert oder sogar perhalogeniert ist,
wobei die Gruppen R und R' gemeinsam mit N einen 5- oder 6-gliedrigen Kohlenstoffring bilden können, der auch mindestens ein Heteroatom im Ring enthalten kann, das unter O, N und/oder S ausgewählt ist, und/oder mit 1 bis 5 gleichen oder verschiedenen Gruppen substituiert sein kann, die unter -OH; -OR"; -O-COR"; -SH; -SR"; -S-COR"; -NH₂; -NHR"; -NH-COR"; -Hal (Halogen); -CN; -COOR"; -COR" ausgewählt sind, wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist;
- die Gruppe X eine Gruppe, die unter -OH, -OR₄, -NH₂, -NHR₄, -NR₄R₅, -SR₄, -COOR₄, -COR₄ ausgewählt ist,
wobei R₄ und R₅ unabhängig voneinander eine geradkettige, cyclische oder verzweigte, gesättigte oder ungesättigte Kohlenwasserstoffgruppe mit 1 bis 6 Kohlenstoffatomen ist, die gegebenenfalls mit 1 bis 5 gleichen oder verschiedenen Gruppen substituiert ist, die unter -OH; -OR; -O-COR; -SH; -SR;
-S-COR; -NH₂; -NHR; -NRR'; -NH-COR; -Hal (Halogen oder sogar Perhalogen); -CN; -COOR; -COR ausgewählt sind,
wobei R und R' unabhängig voneinander eine geradkettige, verzweigte oder cyclische, gesättigte oder ungesättigte Kohlenwasserstoffgruppe mit 1 bis 6 Kohlenstoffatomen bedeuten, die gegebenenfalls halogeniert oder sogar perhalogeniert ist, wobei die Gruppen R und R' gemeinsam mit N einen 5- oder 6-gliedrigen Kohlenstoffring bilden können, der auch mindestens ein Heteroatom im Ring enthalten kann, das unter O, N und/oder S ausgewählt ist, und/oder mit 1 bis 5 gleichen oder verschiedenen Gruppen substituiert sein kann, die unter -OH; -OR"; -O-COR"; -SH; -SR"; -S-COR"; -NH₂; -NHR"; -NH-COR"; -Hal (Halogen); -CN; -COOR"; -COR" ausgewählt sind, wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist;
wobei die Gruppen R₄ und R₅ gemeinsam mit N einen 5- oder 6-gliedrigen Kohlenstoffring bilden können, der ferner mindestens ein Heteroatom im Ring enthalten kann, das unter O, N und/oder S ausgewählt ist, und/oder mit 1 bis 5 gleichen oder verschiedenen Gruppen substituiert sein kann, die unter -OH; -OR"; -O-COR"; -SH; -SR"; -S-COR"; -NH₂; -NHR"; -NH-COR"; -Hal (Halogen); -CN; -COOR"; -COR" ausgewählt sind, wobei R" eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist,
die Salze dieser Verbindungen mit organischen oder anorganischen Säuren und ihre optischen Isomere in isolierter Form oder als racemisches Gemisch,
wobei die folgenden Derivate ausgenommen sind:
- N-Acetylglycyl-N-(benzyl)-DL-valylglycin-*t*-butylester,
- N-Acetylglycyl-N-(2-nitrobenzyl)-DL-valylglycin-*t*-butylester,
- N-Acetylglycyl-N-(2,4-dimethyloxybenzyl)-DL-valylglycin-*t*butylester.

2. Verbindung nach Anspruch 1, wobei:
- die Gruppe Y Sauerstoff bedeutet, und/oder
- die Gruppe R₁ Wasserstoff oder eine geradkettige oder verzweigte, gesättigte oder ungesättigte Kohlenwasserstoffgruppe mit 1 bis 12 Kohlenstoffatomen und insbesondere 1, 2, 3, 4, 5 oder 6 Kohlenstoffatomen bedeutet, die gegebenenfalls substituiert ist, und/oder
- die Substituenten von R₁ unter -OH, -OR und/oder -P(O)-(OR)₂ ausgewählt sind, wobei R eine geradkettige, verzweigte oder cyclische, gesättigte oder ungesättigte Kohlenwasserstoffgruppe mit 1 bis 6 Kohlenstoffatomen ist, die gegebenenfalls halogeniert oder sogar perhalogeniert vorliegt; und/ oder
- die Gruppe R₂ eine geradkettige verzweigte oder cyclische, gesättigte oder ungesättigte Kohlenwasserstoffgruppe mit 1 bis 12 Kohlenstoffatomen und insbesondere 1, 2, 3, 4, 5 oder 6 Kohlenstoffatomen ist, die gegebenenfalls substituiert vorliegt; und/oder
- die Substituenten von R₂ unter -OH und -OR ausgewählt sind,
wobei R eine geradkettige, verzweigte oder cyclische, gesättigte oder ungesättigte Kohlenwasserstoffgruppe mit 1 bis 6 Kohlenstoffatomen ist, die gegebenenfalls halogeniert oder sogar perhalogeniert vorliegt; und/oder
- die Gruppe R₃ eine Gruppe der Formel -C₆H_{(5-y')}-B_{y'} mit y' = 1, 2 oder 3; oder eine Gruppe der Formel -A-C₆H_{(5-y)}-B_{y} mit y = 0, 1 oder 2 bedeutet; und/oder
- die Gruppe A von R₃ eine zweiwertige, geradkettige oder verzweigte, gesättigte oder ungesättigte Kohlenwasserstoffgruppe mit 1 bis 12 Kohlenstoffatomen ist, die gegebenenfalls substituiert ist; und/oder
- die Gruppe B von R₃ mindestens eine Gruppe -OR; -NHR; -CN;
- COOR; -COR oder eine geradkettige oder verzweigte, gesättigte oder ungesättigte Kohlenwasserstoffgruppe mit 1 bis 12 Kohlenstoffatomen bedeutet, die gegebenenfalls substituiert ist; und/oder
- die Substituenten von A und/oder B unter -Hal (Halogen oder sogar Perhalogen); -CN; -COOR; -NO₂; -SO₂-OR ausgewählt sind, wobei R eine geradkettige, verzweigte oder cyclische, gesättigte oder ungesättigte Kohlenwasserstoffgruppe mit 1 bis 6 Kohlenstoffatomen bedeutet, die gegebenenfalls halogeniert oder sogar perhalogeniert ist; und/ oder
- die Gruppe X eine Gruppe bedeutet, die unter -OH oder -OR₄ ausgewählt ist, wobei R₄ eine geradkettige, cyclische oder verzweigte, gesättigte oder ungesättigte Kohlenwasserstoffgruppe mit 1 bis 6 Kohlenstoffatomen bedeutet, die gegebenenfalls substituiert ist; und/oder
- die Substituenten von R₄ in X unter -OH und -OR ausgewählt sind, wobei R eine geradkettige, verzweigte oder cyclische, gesättigte oder ungesättigte Kohlenwasserstoffgruppe mit 1 bis 6 Kohlenstoffatomen ist, die gegebenenfalls halogeniert oder sogar perhalogeniert ist.

3. Verbindung nach einem der vorhergehenden Ansprüche, wobei:
- die Gruppe R₁ eine Gruppe Methyl, Ethyl, Propyl oder Isopropyl bedeutet, die gegebenenfalls mit einer Gruppe -OH oder -P(O)-(OR)₂ substituiert ist, wobei R Methyl, Ethyl, Propyl oder Isopropyl bedeutet; und/oder
- die Gruppe R₂ eine Gruppe Methyl, Ethyl, Propyl, Isopropyl, n-Butyl, *t*-Butyl oder Isobutyl bedeutet; und/oder
- die Gruppe R₃ eine Gruppe bedeutet, die unter den Gruppen der folgenden Formeln ausgewählt ist: 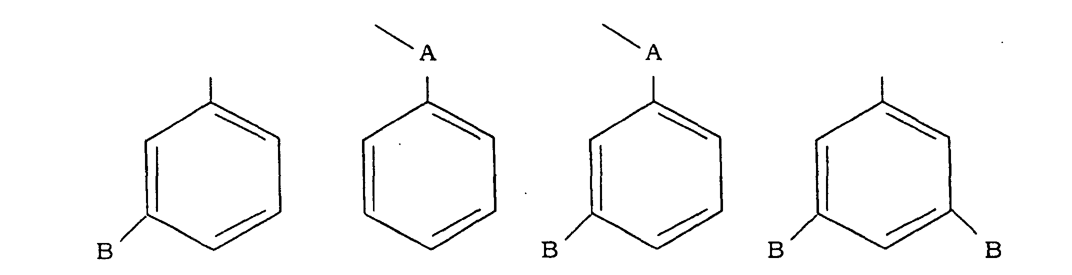 worin die zweiwertige Gruppe A Methylen, Ethylen, Propylen bedeutet und/oder die Gruppe B mindestens eine Gruppe -OR; -NHR; -CN; -COOR; -COR bedeutet, worin die Gruppen R Methyl, Ethyl, Propyl oder Isopropyl bedeutet, oder eine Kohlenwasserstoffgruppe, die unter Methyl, Ethyl, Propyl oder Isopropyl ausgewählt ist und mit einem oder mehreren Halogenen, insbesondere Chlor, Brom, Iod oder Fluor substituiert und vorzugsweise vollständig halogeniert (perhalogeniert), beispielsweise perfluoriert ist, und insbesondere die Perfluormethylgruppe (-CF₃) bedeutet;
- die Gruppe X eine Gruppe bedeutet, die unter -OH, -OCH₃, -OC₂H₅, -O-C₃H₇ oder -OC₄H₉ ausgewählt ist.

4. Verbindung nach einem der vorhergehenden Ansprüche mit der chemischen Bezeichnung:
- {2-[Acetyl-(3-trifluormethyl-phenyl)-amino]-3-methyl-butyrylamino}-essigsäure
- Ethyl-{2-(acetyl-(3-trifluormethyl-phenyl)-amino]-3-methylbutyrylamino}-acetat
- [2-(Acetyl-benzyl-amino)-3-methyl-butyrylamino]-essigsäure,
- Ethyl-[2-(acetyl-benzyl-amino)-3-methyl-butyrylamino]-acetat,
- Ethyl-(2-{-benzyl-[(diethoxy-phosphoryl)-acetyl]-amino}-3-methyl-butyrylamino)-acetat.

5. Zusammensetzung, die in einem physiologisch akzeptablen Medium mindestens eine Verbindung der Formel (I) nach einem der Ansprüche 1 bis 4 enthält.

6. Zusammensetzung nach Anspruch 5, wobei die Verbindung der Formel (I) in einer Menge von 0,00001 bis 20 Gew.-%, bezogen auf das Gesamtgewicht der Zusammensetzung, insbesondere 0,001 bis 10 Gew.-%, vorzugsweise 0,05 bis 5 Gew.-%, noch besser 0,1 bis 2 Gew.-% und bevorzugt 0,5 bis 1 Gew.-% enthalten ist.

7. Zusammensetzung nach einem der Ansprüche 5 bis 6, die als kosmetische oder pharmazeutische Zusammensetzung vorliegt, die zur Pflege und/oder zur Behandlung von ulzerierten Bereichen oder Bereichen vorgesehen sind, die Stress oder Mikrostress ausgesetzt waren, der insbesondere durch UV-Exposition und/oder Kontakt mit einem reizenden Produkt ausgelöst wurde.

8. Zusammensetzung nach einem der Ansprüche 5 bis 6, die in den folgenden Formen vorliegt:
- als Produkt zur Pflege, zur Behandlung, zur Reinigung oder zum Schutz der Haut des Gesichts oder des Körpers einschließlich der Kopfhaut, beispielsweise als pflegende Zusammensetzung (Tagesprodukt, Nachtprodukt, hydratisierendes Produkt) für das Gesicht oder den Körper; Zusammensetzung gegen Falten oder Anti-Age-Zusammensetzung für das Gesicht; mattierende Zusammensetzung für das Gesicht; Zusammensetzung gegen Hautirritationen;
- Zusammensetzung zum Sonnenschutz, zur künstlichen Bräunung (Selbstbräunung) oder zur Pflege nach der Sonnenexposition;
- Zusammensetzung für die Haarbehandlung, insbesondere Creme oder Gel zum Sonnenschutz; Zusammensetzung zur Pflege der Kopfhaut, insbesondere gegen Haarausfall oder für den Haarwuchs;
- Produkt zum Schminken der Gesichtshaut, der Haut des Körpers oder der Lippen, beispielsweise Make-up, getönte Creme, Wangenrouge, Lidschatten, loser oder kompaktierter Puder, Stift zum Abdecken von Augenringen, Abdeckstift, Lippenstift, Lippenpflegeprodukt;
- Hygieneprodukt (buccal), beispielsweise Zahncreme oder Mundspüllotion.

9. Zusammensetzung nach einem der Ansprüche 5 bis 8, die als Zusammensetzung zur Pflege der Haut des Gesichts vom Typ Antifalten-Produkt oder Anti-Age-Produkt oder als Zusammensetzung zum Sonnenschutz oder After-Sun-Produkt vorliegt.

10. Verwendung mindestens einer Verbindung der Formel (I) nach einem der Ansprüche 1 bis 4 oder einer sie enthaltenden Zusammensetzung nach einem der Ansprüche 5 bis 9 zur Herstellung eines Arzneimittels, das dazu vorgesehen ist, präventiv oder kurativ die Anzeichen der Haltalterung am Körper oder im Gesicht, die lichtinduziert oder altersbedingt sein kann, und insbesondere die Alterung zu bekämpfen, die durch eine Verminderung der Elastizität der Haut und/oder eine Zerstörung des Collagens in den Gewebestrukturen verursacht wird.

11. Verwendung mindestens einer Verbindung der Formel (I) nach einem der Ansprüche 1 bis 4 oder einer die Verbindung enthaltenden Zusammensetzung, nach einem der Ansprüche 5 bis 9 zur Herstellung eines Arzneimittels, das dazu vorgesehen ist, präventiv oder kurativ die Falten und/oder Fältchen, welke Haut, Mangel an Elastizität oder Tonus der Haut, Dünnerwerden der Dermis, Zerstörung von Collagenfasern, weiche Haut, dünne Haut; und innere Schäden der Haut, die von einer Exposition gegenüber UV-Strahlung herrühren, zu behandeln.

12. Verwendung mindestens einer Verbindung nach einem der Ansprüche 1 bis 4 oder einer sie enthaltenden Zusammensetzung, nach einem der Ansprüche 5 bis 9 zur Herstellung eines Arzneimittels, das dazu vorgesehen ist, die Aktivität der Elastasen zu inhibieren und/oder die Zerstörung von elastischen Fasern zu begrenzen und/oder zu bekämpfen.

13. Verfahren zur kosmetischen Behandlung der Haut des Körpers oder des Gesichts einschließlich der Kopfhaut, wobei auf die Haut eine kosmetische Zusammensetzung nach einem der Ansprüche 5 bis 9 aufgetragen wird.

## Claims

1. Compound of formula (I): 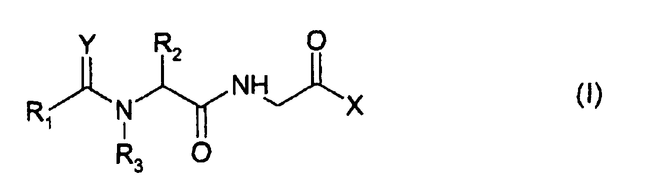 in which:
- the radical Y represents O or S,
- the radical R1 represents:
- (i) a hydrogen atom;
- (ii) a linear, branched or cyclic, saturated or unsaturated hydrocarbon-based radical containing 1 to 18 carbon atoms,
optionally substituted with 1 to 5 groups, which may be identical or different, chosen from -OH; -OR; -O-COR; -SH; -SR; -S-COR; -NH₂; -NHR; -NRR'; -NH-COR; -Hal (halogen); -CN; -COOR; -COR; -P(O)-(OR)₂; -SO₂-OR;
with R and R' representing, independently of each other, a linear, branched or cyclic, saturated or unsaturated hydrocarbon-based radical containing 1 to 6 carbon atoms, which is optionally halogenated, or even perhalogenated,
the said radicals R and R' possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR" ; -SH; -SR" ; -S-COR" ; -NH₂; -NHR" ; -NH-COR" ; -Hal (halogen); -CN; -COOR"; -COR''; with R'' representing a linear, branched or cyclic, saturated or unsaturated hydrocarbon-based radical containing 1 to 6 carbon atoms, which is optionally halogenated, or even perhalogenated;
- (iii) a radical chosen from the following radicals: -OR; -NH₂; -NHR; -NRR'; -NH-COR; -COOR; -COR;
with R and R' representing, independently of each other, a linear, branched or cyclic, saturated or unsaturated hydrocarbon-based radical containing 1 to 6 carbon atoms, which is optionally halogenated, or even perhalogenated,
the said radicals R and R' possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR''; -SH;
-SR" ; -S-COR" ; -NH₂; -NHR" ; -NH-COR''; -Hal (halogen); -CN; -COOR''; -COR"; with R" representing a linear, branched or cyclic, saturated or unsaturated hydrocarbon-based radical containing 1 to 6 carbon atoms, which is optionally halogenated, or even perhalogenated;
- the radical R2 represents a linear, branched or cyclic, saturated or unsaturated hydrocarbon-based radical containing 1 to 18 carbon atoms,
optionally substituted with 1 to 5 groups, which may be identical or different, chosen from -OH; -OR; -O-COR; -SH; -SR; -S-COR; -NH₂; -NHR; -NRR'; -NH-COR; -Hal (halogen); -CN; -COOR; -COR;
with R and R' representing, independently of each other, a linear, branched or cyclic, saturated or unsaturated hydrocarbon-based radical containing 1 to 6 carbon atoms, which is optionally halogenated, or even perhalogenated,
the said radicals R and R' possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR''; -SH; -SR"; -S-COR''; -NH₂; -NHR"; -NH-COR" ; -Hal (halogen); -CN; -COOR"; -COR''; with R" representing a linear, branched or cyclic, saturated or unsaturated hydrocarbon-based radical containing 1 to 6 carbon atoms, which is optionally halogenated, or even perhalogenated;
- the radical R3 represents a radical chosen from those of formula (II) or (III):
(II) -A-C₆H(_{5-y})-B_{y}
(III) -C₆H(_{5-y'})-B_{y'}
in which:
- y is an integer between 0 and 5 inclusive and y' is an integer between 1 and 5 inclusive;
- A is a linear or branched, saturated or unsaturated divalent hydrocarbon-based radical containing 1 to 18 carbon atoms,
optionally substituted with 1 to 5 groups, which may be identical or different, chosen from -OH; -OR; -O-COR; -SH; -SR; -S-COR; -NH₂; -NHR; -NRR'; -NH-COR; -Hal (halogen, or even perhalogen); -CN; -COOR; -COR; -NO₂; -SO₂-OR;
with R and R' representing, independently of each other, a linear, branched or cyclic, saturated or unsaturated hydrocarbon-based radical containing 1 to 6 carbon atoms, which is optionally halogenated, or even perhalogenated,
the said radicals R and R' possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR"; -SH; -SR" ; -S-COR''; -NH₂; -NHR"; -NH-COR''; -Hal (halogen); -CN; -COOR''; -COR"; with R" representing a linear, branched or cyclic, saturated or unsaturated hydrocarbon-based radical containing 1 to 6 carbon atoms, which is optionally halogenated, or even perhalogenated;
- B represents at least one group, which may be identical or different, chosen from -OH; -OR; -O-COR; -SH; -SR; -S-COR; -NH₂; -NHR; -NRR'; -NH-COR; -Halogen; -CN; -COOR; -COR; -NO₂; -SO₂-OR, or represents a linear or branched, saturated or unsaturated hydrocarbon-based radical containing 1 to 18 carbon atoms,
optionally substituted with 1 to 5 groups, which may be identical or different, chosen from -OH; -OR; -O-COR; -SH; -SR; -S-COR; -NH₂; -NHR; -NRR'; -NH-COR; -Hal (halogen, or even perhalogen); -CN; -COOR; -COR; -NO₂; -SO₂-OR;
with R and R' representing, independently of each other, a linear, branched or cyclic, saturated or unsaturated hydrocarbon-based radical containing 1 to 6 carbon atoms, which is optionally halogenated, or even perhalogenated,
the said radicals R and R' possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR" ; -SH; -SR" ; -S-COR''; -NH₂; -NHR" ; -NH-COR''; -Hal (halogen); -CN; -COOR"; -COR"; with R" representing a linear, branched or cyclic, saturated or unsaturated hydrocarbon-based radical containing 1 to 6 carbon atoms, which is optionally halogenated, or even perhalogenated;
- the radical X represents a radical chosen from -OH, -OR₄, -NH₂, -NHR₄, -NR₄R₅, -SR₄, -COOR₄ and -COR₄;
with R₄ and R₅ representing, independently of each other, a linear, branched or cyclic, saturated or unsaturated hydrocarbon-based radical containing 1 to 6 carbon atoms, optionally substituted with 1 to 5 groups, which may be identical or different, chosen from -OH; -OR; -O-COR; -SH; -SR; -S-COR; -NH₂; -NHR; -NRR'; -NH-COR; -Hal (halogen, or even perhalogen); -CN; -COOR; -COR; with R and R' representing, independently of each other, a linear, branched or cyclic, saturated or unsaturated hydrocarbon-based radical containing 1 to 6 carbon atoms, which is optionally halogenated, or even perhalogenated;
the said radicals R and R' possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR''; -SH; -SR"; -S-COR''; -NH₂; -NHR"; -NH-COR''; -Hal (halogen); -CN; -COOR''; -COR''; with R'' representing a linear, branched or cyclic, saturated or unsaturated hydrocarbon-based radical containing 1 to 6 carbon atoms, which is optionally halogenated, or even perhalogenated;
the said radicals R₄ and R₅ possibly forming together with N a 5- or 6-membered carbon-based ring which may also comprise at least one hetero atom chosen from O, N and/or S in the ring, and/or which may be substituted with 1 to 5 groups, which may be identical or different, chosen from -OH; -OR''; -O-COR''; -SH; -SR"; -S-COR''; -NH₂; -NHR"; -NH-COR''; -Hal (halogen); -CN; -COOR''; -COR''; with R'' representing a linear, branched or cyclic, saturated or unsaturated hydrocarbon-based radical containing 1 to 6 carbon atoms, which is optionally halogenated, or even perhalogenated;
the mineral acid or organic acid salts thereof and the optical isomers thereof, in isolated form or as a racemic mixture,
with the exception of the following derivatives:
- *N*-acetylglycyl-*N*-(benzyl)-DL-valylglycine tert-butyl ester;
- *N*-acetylglycyl-*N*-(2-nitrobenzyl)-DL-valylglycine *tert*-butyl ester;
- *N*-acetylglycyl-*N*-(2,4-dimethyloxybenzyl)-DL-valylglycine *tert*-butyl ester.

2. Compound according to Claim 1, in which:
- the radical Y represents oxygen, and/or
- the radical R1 represents hydrogen or a linear or branched, saturated or unsaturated, optionally substituted hydrocarbon-based radical containing 1 to 12 and in particular 1, 2, 3, 4, 5 or 6 carbon atoms, and/or
- the substituents of R1 are chosen from -OH, -OR and/or -P(O)-(OR)₂ with R representing a linear, branched or cyclic, saturated or unsaturated hydrocarbon-based radical containing 1 to 6 carbon atoms, which is optionally halogenated, or even perhalogenated; and/or
- the radical R2 represents a linear, branched or cyclic, saturated or unsaturated, optionally substituted hydrocarbon-based radical containing 1 to 12 and in particular 1, 2, 3, 4, 5 or 6 carbon atoms; and/or
- the substituents of R2 are chosen from -OH and -OR with R representing a linear, branched or cyclic, saturated or unsaturated hydrocarbon-based radical containing 1 to 6 carbon atoms, which is optionally halogenated, or even perhalogenated; and/or
- the radical R3 represents a radical of formula
- C₆H_{(5-y')}-B_{y'} for which y' = 1, 2 or 3; or a radical of formula -A-C₆H_{(5-y)}-B_{y} for which y = 0, 1 or 2; and/or
- the radical A of R3 is a linear or branched, saturated or unsaturated, optionally substituted divalent hydrocarbon-based radical containing 1 to 12 carbon atoms; and/or
- the radical B of R3 represents at least one group
- OR; -NHR; -CN; -COOR; -COR or represents a linear or branched, saturated or unsaturated, optionally substituted hydrocarbon-based radical containing 1 to 12 carbon atoms; and/or
- the substituents of A and/or of B are chosen from
- Hal (halogen, or even perhalogen); -CN; -COOR; -NO₂;
- SO₂-OR; with R representing a linear, branched or cyclic, saturated or unsaturated hydrocarbon-based radical containing 1 to 6 carbon atoms, which is optionally halogenated, or even perhalogenated; and/or
- the radical X represents a radical chosen from -OH or
- OR₄ with R₄ representing a linear, cyclic or branched, saturated or unsaturated hydrocarbon-based radical containing 1 to 6 carbon atoms, which is optionally substituted; and/or
- the substituents of R₄ of X are chosen from -OH and
- OR with R representing a linear, branched or cyclic, saturated or unsaturated hydrocarbon-based radical containing 1 to 6 carbon atoms, which is optionally halogenated, or even perhalogenated.

3. Compound according to either of the preceding claims, in which:
- the radical R1 represents a methyl, ethyl, propyl or isopropyl radical, optionally substituted with a group
- OH or -P(O)-(OR)₂ with R representing methyl, ethyl, propyl or isopropyl; and/or
- the radical R2 represents a methyl, ethyl, propyl, isopropyl, n-butyl, tert-butyl or isobutyl radical; and/or
- the radical R3 represents a group chosen from one of the following formulae: 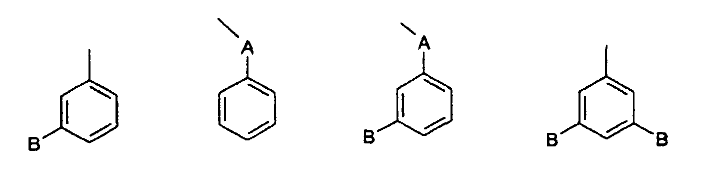
in which the divalent radical A is a methylene, an ethylene or a propylene and/or the radical B represents at least one group -OR; -NHR; -CN; -COOR; -COR for which R denotes a methyl, ethyl, propyl or isopropyl radical or represents a hydrocarbon-based radical chosen from a methyl, ethyl, propyl or isopropyl radical, substituted with one or more halogens, in particular chlorine, bromine, iodine or fluorine, and preferably totally halogenated (perhalogenated), such as perfluorinated, in particular the perfluoromethyl radical (-CF₃) ,
- the radical X represents a radical chosen from -OH,
- OCH₃, -OC₂H₅, -O-C₃H₇ and -OC₄H₉.

4. Compound according to one of the preceding claims, the chemical name of which is:
- {2-[acetyl(3-trifluoromethylphenyl)amino]-3-methylbutyrylamino}acetic acid,
- ethyl {2-[acetyl(3-trifluoromethylphenyl)amino]-3-methylbutyrylamino}acetate,
- [2-(acetylbenzylamino)-3-methylbutyrylamino]acetic acid,
- ethyl [2-(acetylbenzylamino)-3-methylbutyrylamino]-acetate,
- ethyl (2-{benzyl[(diethoxyphosphoryl)acetyl]amino}-3-methylbutyrylamino)acetate.

5. Composition comprising, in a physiologically acceptable medium, at least one compound of formula (I) as defined in one of Claims 1 to 4.

6. Composition according to Claim 5, in which the compound of formula (I) is present in an amount of between 0.00001% and 20% by weight relative to the total weight of the composition, in particular between 0.001% and 10% by weight, preferably between 0.05% and 5% by weight, better still between 0.1% and 2% by weight and preferentially between 0.5% and 1% by weight.

7. Composition according to either of Claims 5 and 6, which is in the form of a cosmetic or pharmaceutical composition intended to care for and/or treat ulcerated areas or areas which have suffered cutaneous stress or microstress, brought about in particular by exposure to UV and/or by coming into contact with an irritant product.

8. Composition according to either of Claims 5 and 6, which is in the form:
- of a care, treatment, cleansing or protective product for facial or body skin, including the scalp, such as a care composition (moisturizing day or night composition) for the face or the body; an anti-wrinkle or anti-ageing composition for the face; a matt-effect composition for the face; a composition for irritated skin;
- of an anti-sun composition, an artificial tanning (self-tanning) composition or an after-sun care composition;
- of a haircare composition, and in particular an anti-sun cream or gel; a scalp care composition, in particular to prevent hair loss or to promote regrowth of the hair;
- of a make-up product for the skin of the face, the body or the lips, such as a foundation, a tinted cream, a blusher, an eyeshadow, a free or compact powder, a concealer stick, a cover stick, a lipstick or a lipcare product;
- of an oral hygiene product such as a toothpaste or an oral rinsing lotion.

9. Composition according to one of Claims 5 to 8, which is in the form of a facial skincare composition, of anti-wrinkle or anti-ageing type, or of an anti-sun or after-sun composition.

10. Use of at least one compound of formula (I) as defined in one of Claims 1 to 4, or of a composition comprising it as defined in one of Claims 5 to 9, for the preparation of a medicinal product intended to treat, preventively or curatively, the signs of ageing of facial or body skin, whether chronobiological or light-induced ageing, and in particular the ageing generated by a reduction in the elasticity of the skin and/or by a degradation of the collagen in the structure of the tissues.

11. Use of at least one compound of formula (I) as defined in one of Claims 1 to 4, or of a composition comprising it as defined in one of Claims 5 to 9, for the preparation of a medicinal product intended to treat, preventively or curatively, wrinkles and/or fine lines, wizened skin, lack of elasticity and/or of tonus of the skin, thinning of the dermis, the degradation of the collagen fibres, flaccid skin and thinned skin; the internal degradation of the skin following exposure to ultraviolet radiation.

12. Use of at least one compound of formula (I) as defined in one of Claims 1 to 4, or of a composition comprising it as defined in one of Claims 5 to 9, for the preparation of a medicinal product intended to inhibit the activity of elastases and/or to limit and/or combat the degradation of the elastic fibres.

13. Cosmetic treatment process for facial or body skin, including the scalp, in which a cosmetic composition as defined in one of Claims 5 to 9 is applied to the skin.
